# EUROPEAN PATENT APPLICATION

(11) **EP 4 079 409 A1**
(43) Date of publication of application: **26.10.2022**
(21) Application number: 21382356.0
(22) Date of filing: 23.04.2021
(51) Int. Cl.: B04B 15/06, B04B 15/02, A61L 2/06, A61L 2/24

(54) **AUTOMATIC SELF-STERILIZING SUPERCENTRIFUGE APPARATUS AND AUTOMATIC SELF-STERILIZING METHOD**

(71) Applicant: Riera Nadeu, S.A., 08403 Granollers (Barcelona) (ES)
(72) Inventor: RIERA DOMÈNECH, Marc, 08017 Barcelona (ES)
(74) Representative: Juncosa Miró, Jaime

(57) **Abstract**

Automatic self-sterilizing supercentrifuge apparatus including a tubular rotor (10) defining a clarification chamber (1); a sealed housing (20) containing said tubular rotor (10), defining an intermediate chamber (2); a cleaning system (30) and a cleaning liquid evacuation system (40); a sterilizing system (50) configured to generate a sterilizing fluid (F2) to sterilize the intermediate chamber (2) and the clarification chamber (1), and a sterilizing fluid evacuation system (60) to evacuate the sterilizing fluid (F2); wherein the sterilizing system (50) includes at least one air sterilization unit (51), configured to generate dry sterilized air (F1) with a moisture content below at least 10% and at least one air heater (52) configured to heat the dry sterilized air to at least 120°C to produce heated sterilized air as the sterilizing fluid (F2), at a pressure above the ambient pressure and below a pressure requiring a certificated pressure-containment-equipment by regulations.

## Description

### Technical field

This invention relates to an automatic self-sterilizing supercentrifuge apparatus which includes a tubular rotor for performing an operation known as liquid clarification, which consists in the settling, by centrifugal force, of a solid fraction from a liquid fraction, said solid fraction being made of solid particles suspended in the liquid fraction, the liquid fraction containing one or two non-miscible liquids. This operation is typically performed to separate different fractions of the human blood.

By supercentrifuge is meant a centrifuge operating at speeds between 15,000 and 30,000g, preferably between 15,000 and 20,000g.

Liquid fractions are automatically extracted from the tubular rotor during its operation, and the solid fraction is periodically extracted from the tubular rotor in an automated manner, while the tubular rotor is not rotating. The supercentrifuge, once empty, shall be cleaned and sterilized before a new batch is treated.

The present invention is also directed to an automatic self-sterilizing method.

### State of the Art

Automatic self-sterilizing supercentrifuge apparatus are already known, for example through documents US2011190111A1, US2004157718A1 or JP2006021121A.

Some of those documents describe the use of a sterilizing liquid to sterilize the interior of the apparatus, but this system requires the use of a large quantity of sterilizing liquid, which is expensive and a complex waste to manage.

Other documents describe the use of steam as sterilizing fluid, but the steam produces condensation within the apparatus, which has to be drained and dried maintaining the sterilization of the apparatus before it is newly used. Furthermore, the steam tends to produce a lot of pressure in internal parts of the apparatus, requiring special sealings and bearings, which are very expensive and prone to fail, requiring a very robust and expensive housing.

The present invention proposes a solution to the above and other problems.

### Brief description of the invention

The present invention is directed, according to a first aspect, towards an automatic self-sterilizing supercentrifuge apparatus for liquid clarification.

A supercentrifuge is a centrifuge operating at speeds between 15,000 and 30,000g, preferably between 15,000 and 20,000g. The present invention can also be used in centrifuges with lower speeds.

The liquid clarification consists in the settling, by centrifugal force, of a solid fraction suspended in a liquid fraction, the liquid fraction containing one liquid or two non-miscible liquids.

The proposed automatic self-sterilizing supercentrifuge apparatus includes, in a manner already known in the state of the art, the following elements:
Automatic self-sterilizing supercentrifuge apparatus for liquid clarification including:
- a tubular rotor actuated by an actuation motor to rotate around a vertical axis, said tubular rotor including a hollow interior defining a clarification chamber;
- a sealed housing containing said tubular rotor, defining an intermediate chamber between the tubular rotor and the housing;
- a cleaning system including a cleaning liquid source connected to the intermediate chamber and to the clarification chamber, and a cleaning liquid evacuation system connected to the intermediate chamber and to the clarification chamber for evacuation of a cleaning liquid thereof;
- a sterilizing system in communication with the intermediate chamber and with the clarification chamber and configured to generate a sterilizing fluid to sterilize the intermediate chamber and the clarification chamber, and
- a sterilizing fluid evacuation system in communication with the intermediate chamber and with the clarification chamber and configured to evacuate the sterilizing fluid thereof.

According to that, the tubular rotor is a hollow rotor contained within a hollow static housing. Said tubular rotor is connected to the housing through bearings allowing the rotation thereof around a vertical axis, driven by an actuation motor connected thereto.

The tubular rotor is hollow and defines a clarification chamber. Said clarification chamber can be accessible from the outside of the tubular rotor and optionally from the outside of the housing through an upper connection and a lower connection, which are preferably concentric with the vertical axis, and placed on opposed ends of the tubular rotor. Different fluids can be introduced or extracted from the tubular rotor through said upper and lower connections, on different operational stages of the apparatus.

During a clarification stage a liquid to be clarified is introduced within the clarification chamber, typically through the lower connection, while the tubular rotor rotates. The liquid to be clarified is retained against the inside walls of the tubular rotor by centrifugal force, and a solid fraction of said liquid is settled and accumulated against said inside walls, producing a liquid fraction. The liquid fraction is evacuated from the clarification chamber, typically through the upper connection.

The tubular rotor typically further comprises a fluid driven piston tightly fitted within the tubular rotor. The driven piston can include through holes with valves for allowing the evacuation of the liquid fraction during the clarification stage and for closing said through holes permitting the pressurization of an upper portion of the clarification chamber, above the piston, with a fluid such sterilized air introduced therein through the upper connection, producing a descendent movement of the piston, preferably when the tubular rotor is not rotating. This descendent movement of the piston pushes the solid fraction out of the clarification chamber through the lower connection.

Once the solid and liquid fractions have been removed from the clarification chamber, the apparatus has to be cleaned and sterilized before a new clarification stage starts.

During a cleaning stage an automatic cleaning is produced by introducing a cleaning liquid provided by a cleaning liquid source, such clean water or purified water, in the clarification chamber through the upper or the lower connections, preferably when the tubular rotor is rotating, and in the intermediate chamber, preferably through nozzles, such sprinklers, contained in the intermediate chamber and oriented towards the exterior surfaces of the tubular rotor. The cleaning stage can last for approximately 20 minutes (± 5 min).

The cleaning system further includes a cleaning liquid evacuation system which evacuates the cleaning liquid from the clarification chamber and from the intermediate chamber, preferably through the bottom end of those chambers.

During a sterilization stage a sterilizing fluid source provides a sterilizing fluid to the clarification chamber and to the intermediate chamber to perform an automatic sterilization thereof. The sterilization fluid introduced in the clarification chamber and in the intermediate chamber is later extracted from those chambers by a sterilizing fluid evacuation system.

The present invention proposes, in a manner not known from the state of the art documents, the inclusion, in the sterilizing fluid source, of at least one air sterilization unit, configured to generate dry sterilized air with a moisture content below at least 10%, or below 5% or more preferably equal or below 1%, and at least one air heater configured to heat the dry sterilized air to at least 120°C to produce heated sterilized air as the sterilizing fluid.

The sterilizing fluid above 120°C is sufficient to produce an effective sterilization of the machine if said sterilizing fluid is maintained within the machine during sufficient time, typically longer than five hours.

The sterilization time can be effectively reduced by increasing the temperature of the sterilizing fluid. For example, the sterilization time will be of around three hours with a sterilizing fluid at 140°C, around two hours with a sterilizing fluid at 160°C, around one hour with the sterilizing fluid at 170°C and only half an hour with the sterilizing fluid at 180°C.

The sterilizing system is configured to provide the sterilizing fluid to the intermediate chamber and to the clarification chamber at a pressure comprised between a minimal working pressure, defined above the ambient pressure, and a maximal working pressure defined below a pressure requiring a certificated pressure-containment-equipment by regulations.

The use of a sterilizing fluid with a pressure above the ambient pressure ensures that the ambient air, which is not sterile, does not enter into the supercentrifuge through bearing leakages or seal leakages. But as higher the pressure is, the higher the risk of a breakage, disconnection, tearing or explosion of some elements or pipes of the supercentrifuge, therefore regulations define a maximal working pressure for equipment and above said maximal working pressure a special certification of the equipment is required to ensure its safety.

Because the pressure of the sterilizing fluid is above the ambient pressure and below the pressure requiring certificated pressure-containment-equipment, the bearings and seals can be simple and cheap, and the vessels, pipes and their connections can also be simple, cheap, and light in its construction, the proposed supercentrifuge being a non-certified equipment.

A pressure requiring a certificated pressure-containment-equipment is defined by regulations such as the Pressure Equipment Directive (PED) (2014/68/EU) or other equivalent regulations. Above said pressure the equipment containing a fluid with such pressure has to be officially certificated to ensure that an explosion risk is avoided.

A sterilizing fluid with a pressure equal or above the pressure requiring a certificated pressure-containment-equipment require the use of a stronger and heavier sealed housing, a more expensive pipes, seals, bearings, valves, etc. and all this equipment shall be certified. This makes the equipment expensive and heavy and its maintenance also more expensive. Maintaining the maximal working pressure below said pressure makes the equipment lighter and cheaper.

Typically said pressure requiring a certificated pressure-containment-equipment is a pressure equal or above 500mbars above the ambient pressure, but other values can be defined in different regulations.

In any case, it is preferred to define a maximal working pressure below 500mbars above the ambient pressure, preferably below 200mbars above the ambient pressure or 100mbars above the ambient pressure.

Maintaining the pressure above the ambient pressure also ensures that the entrance of non-sterile ambient air into the intermediate chamber or the clarification chamber is prevented.

The sterilization unit can obtain purified air, free from particles and other pollutants, from an air supply. Preferably, the air supply is configured to provide the air to the at least one air sterilization unit at a pressure above 6 bars. This high pressure permits to perform a sterilization process based on a pressure differential well known in the state of the art.

According to an embodiment of the present invention, the clarification chamber and the intermediate chamber can be in communication to each other permitting the free flow of the sterilizing fluid between said intermediate chamber and said clarification chamber. In this case the sterilizing system is preferably connected to the intermediate chamber and the sterilizing fluid evacuation system is connected at least to the clarification chamber, producing a sterilizing fluid flow from the intermediate chamber to the clarification chamber.

Preferably the clarification chamber is in communication with the intermediate chamber through one end of the clarification chamber, for example the lower end, and the sterilizing fluid evacuation system is connected to an opposed end of the clarification chamber, por example the upper end thereof, producing an ascending flow of sterilizing fluid within the clarification chamber.

Said connection between the intermediate chamber and the clarification chamber allows to use a non-air-tight bearing on the lower end of the rotor, or even to hold the rotor hanging from the upper end, without bearings at its lower end.

Alternatively, it is proposed to connect the sterilizing system directly to the intermediate chamber and to the clarification chamber in an independent manner. The sterilizing fluid evacuation system can be also connected directly to the intermediate chamber and to the clarification chamber in an independent manner. This permits to keep the intermediate chamber and the clarification chamber separated to each other, for example using air-tight bearings between the rotor and the sealed housing.

In any case, the sterilizing fluid evacuation system can include at least one venting valve configured to evacuate sterilizing fluid from the clarification chamber and/or from the intermediate chamber when the pressure exceeds the maximum working pressure defined above as a pressure lower than the pressure requiring a certificated pressure-containment-equipment. This ensures that the pressure is alleviated before reaching the pressure requiring a certificated pressure-containment-equipment, permitting the use of a non-certificated equipment, much simpler and cheaper.

The sterilizing fluid evacuation system can further include an emergency shear valve configured to break under a pressure exceeding the maximum working pressure and lower than the pressure requiring a certificated-pressure-equipment. In case the venting valve does not perform as expected and the pressure raises, the emergency shear valve ensures the alleviation of the pressure before reaching dangerous levels. The emergency shear valve will be configured at a higher pressure than the aperture of the venting valve.

According to another embodiment, the sterilizing fluid evacuation system can be connected to an impeller, configured to suction the sterilizing fluid from the clarification chamber and/or from the intermediate chamber and to reintroduce at least part of said sterilizing fluid within the clarification chamber and/or within the intermediate chamber through a recirculation circuit, producing a flow of sterilizing fluid within the intermediate chamber and/or the clarification chamber.

According to a preferred embodiment, the sterilizing fluid is introduced into the intermediate chamber, and the impeller suctions the sterilizing fluid from the clarification chamber, connected to the intermediate chamber, producing a sterilizing fluid flow from the intermediate chamber to the clarification chamber.

The sterilizing fluid extracted from the clarification chamber is then reintroduced in the intermediate chamber.

The air heater mentioned above can be contained within the intermediate chamber and/or can be in thermal contact with the sealed housing, which in this case will be made of a metallic material with a good thermal conductivity. This configuration produces the heating of the dry sterilized air introduced in the intermediate chamber directly within the intermediate chamber, becoming the sterilizing fluid, in a more efficient manner.

Alternatively, the air heater can be a unit, external to the sealed housing, connected with the air sterilization unit and with the recirculation circuit through electrovalves, said electrovalves being configured to conduct the dry sterilized air produced by the air sterilization unit through the air heater before reaching the intermediate chamber and/or the clarification chamber and to conduct the sterilized fluid flowing through the recirculation circuit through the air heater before reaching the intermediate chamber and/or the clarification chamber.

It is also proposed to include, in the supercentrifuge, a cooling system including a coolant circuit contained within the intermediate chamber and/or in thermal contact with the sealed housing, which in this case will be made of a metallic material with a good thermal conductivity, and/or including a heat sink in the recirculation circuit configured to reduce the temperature of the sterilizing fluid passing there through.

This feature allows accelerating and controlling the cooling cycle of the supercentrifuge to accelerate start of a new clarification cycle.

The coolant circuit can be, for example, a tube through which a coolant fluid flows. The coolant fluid will be preferably water or a water-based fluid.

Because the sterilization cycle reaches temperatures above the water boiling temperature, the coolant circuit will be preferably purged of coolant fluid during the sterilization cycle, for example injecting pressurized air into the coolant circuit to expel the coolant fluid contained therein.

According to that, during a cooling stage the sterilizing fluid evacuation system can be connected, for example through an electro valve, to at least one heat sink for reducing the temperature of the sterilizing fluid evacuated through said sterilizing fluid evacuation system to become a sterilized air at a temperature below 120°C, and preferably at a temperature below 100°C or at an ambient temperature. The at least one heat sink is connected, through an electro valve, to the clarification chamber and/or to the intermediate chamber to recirculate the sterilized air, defining a sterilized recirculation circuit. This sterilized recirculation circuit permits the reduction of the temperature of the apparatus to reach the ambient temperature but using only sterilized air, which has a slight over pressure above the ambient pressure, preventing the entrance of external ambient air.

The cooling stage can last for approximately 40 minutes (± 10 min).

Once the cooling stage has ended, the machine can be maintained for long time periods in a stand-by stage, ensuring that the sterilization is maintained preventing the entrance of non-sterile ambient air by keeping an over-pressure within the machine, waiting for a new use of the apparatus with a low energy consumption. During said stand-by stage the temperature is the ambient temperature and only the pressure has to be maintained by sporadic introduction of new sterilized air when the pressure drops below certain threshold to maintain the pressure slightly above the ambient pressure.

The present invention also proposes that the intermediate chamber contains nozzles oriented towards an exterior surface of the tubular rotor. Said nozzles are connected to the cleaning liquid source to deliver the cleaning fluid within the intermediate chamber through said nozzles.

The sterilizing fluid evacuation system can also include at least one venting valve to evacuate all, or part of the sterilizing fluid evacuated from the clarification chamber and/or from the intermediate chamber. During a drying stage, performed during the initial portion of the sterilization step, the sterilized air, with a temperature below 120°C and preferably with a temperature below 100°C, is introduced in the clarification chamber and in the intermediate chamber to dry the cleaning liquid remaining within said chambers. This drying effect will rise the moisture content of the sterilized air or the sterilizing fluid which can be later vented out and substituted by new sterilized air or new sterilizing fluid with a lower moisture content to continue with the drying process.

The drying stage can last for approximately 10 minutes (± 5 min).

The cleaning liquid source and the liquid evacuation system are preferably connected to opposed ends of the tubular rotor through said lower and upper connections.

The apparatus can further comprise pressure and temperature sensors connected to a control device and said control device being configured to control the pressure and temperature of the sterilized air, the sterilizing fluid and other operational parameters through actuators, such electro valves, following a predefined program and considering information provided by said pressure and temperature sensors.

According to a second aspect, the present invention is directed towards an automatic self-sterilizing method for a supercentrifuge such the one described above.

Said method is applied on a supercentrifuge including at least a tubular rotor, with a hollow interior defining a clarification chamber, and a sealed housing containing said tubular rotor, defining an intermediate chamber between the tubular rotor and the sealed housing.

The proposed method includes the following steps, which are already known in the available state of the art:
- perform a cleaning stage by feeding a cleaning liquid into the intermediate chamber and into the clarification chamber and by evacuating said cleaning liquid from the intermediate chamber and from the clarification chamber;
- perform a sterilizing stage by feeding a sterilizing fluid into the intermediate chamber and into the clarification chamber, keeping the sterilizing fluid within the intermediate chamber and the clarification chamber for a period of time and later evacuating said sterilizing fluid therefrom;

The cleaning stage removes the residues of the clarification process remaining in the clarification chamber and in the intermediate chamber, typically spraying clean water as a cleaning liquid.

During the sterilization stage the sterilizing fluid is introduced in the intermediate chamber and in the clarification chamber, keeping said sterilizing fluid therein for a period of time determined to obtain the desired degree of sterilization of said chambers.

The period of time required to obtain the desired sterilization depends on the temperature of the sterilizing fluid. When the sterilization fluid has a temperature comprised between 120°C and 140°C said period of time will last for between three and six hours, but when the sterilization fluid has a temperature equal or higher than 170°C the period of time required to obtain the desired sterilization will be equal to or lower than an hour. Preferably the temperature will be maintained below 200°C.

The proposed method further includes the following steps, which are not known from the available state of the art:
- during the sterilizing stage the sterilizing fluid is generated by filtering, sterilizing and drying air, producing a dry sterilized air with a moisture content below at least 10%, or below 5% or more preferably equal or below 1%, and by heating said dry sterilized air to a temperature above 120°C, or preferably above 150°C or above 180°C, producing the sterilizing fluid, and said sterilizing fluid is maintained into the clarification chamber and into the intermediate chamber at a pressure comprised between a minimal working pressure, defined above the ambient pressure, and a maximal working pressure defined below a pressure requiring a certificated pressure-containment-equipment.

The sterilization stage can maintain the sterilizing fluid within the intermediate chamber and the clarification chamber at a temperature above 120°C during at least 30 minutes.

Preferably, the sterilizing fluid is introduced in the intermediate chamber and is later suctioned from the clarification chamber, producing a sterilizing fluid flow from the intermediate chamber to the clarification chamber, all or part of the sterilizing fluid being later reintroduced in the intermediate chamber.

According to an embodiment, the sterilization stage includes a cool down stage during which the sealed housing is actively cooled by pumping a coolant through a coolant circuit contained within the intermediate chamber and/or in thermal contact with the sealed housing, which is made of a metallic material, and/or by pumping a coolant through a heat sink in the recirculation circuit configured to reduce the temperature of the sterilizing fluid passing there through.

Preferably the coolant circuit has been drained of coolant before the start of the sterilization stage to prevent the boiling of the coolant and the increase of the pressure within the coolant circuit. Said draining can be achieved, for example, by opening a draining valve in a lower end of the coolant circuit or by injecting pressurized air through the coolant circuit. Preferably the temperature is first reduced by cooling down the temperature of the sterilizing fluid and, once the temperature is below the boiling point of the coolant, the coolant is introduced through the cooling circuit.

After the sterilization stage, a stand-by stage can be performed maintaining the sterilized air within the clarification chamber and the intermediate chamber at the pressure comprised between the minimal working pressure, defined above the ambient pressure, and the maximal working pressure defined below a pressure requiring a certificated pressure-containment-equipment but at ambient temperature, preventing the leakage of the external non-sterile ambient air into the clarification chamber and/or the intermediate chamber with a reduced energy consumption, requiring only occasional introduction of additional sterilized air when the pressure drops below certain pressure threshold close to the minimal working pressure.

It will also be understood that any range of values given may not be optimal in extreme values and may require adaptations of the invention to these extreme values are applicable, such adaptations being within reach of a skilled person.

Other features of the invention appear from the following detailed description of an embodiment.

### Brief description of the Figures

The foregoing and other advantages and features will be more fully understood from the following detailed description of an embodiment with reference to the accompanying drawings, to be taken in an illustrative and non-limitative manner, in which:
Fig. 1 shows a schematic view of the apparatus according to a preferred embodiment, during the clarification stage, wherein the liquid to be clarified is shown as dashed arrows, the clarified liquid fraction is shown as empty arrows and the solid fraction is shown as solid arrows;
Fig. 2 shows the same embodiment shown in Fig. 1 but during the cleaning stage, wherein the cleaning liquid is shown as dashed arrows, when is clean, and as empty arrows when is no longer clean, after the cleaning of the apparatus;
Fig. 3 shows the same embodiment shown in Fig. 1 but during the drying stage, in the initial moments of the sterilization stage, when a drying of the apparatus is performed, wherein the sterilizing fluid is shown as solid arrows;
Fig. 4 shows the same embodiment shown in Fig. 1 but during the sterilization stage, once the drying of the apparatus has been completed, wherein the sterilizing fluid is shown as solid arrows;
Fig. 5 shows the same embodiment shown in Fig. 1 but during the cooling stage, in the final moments of the sterilization stage, once the drying of the apparatus has been completed, wherein the sterilizing fluid is shown as solid arrows;

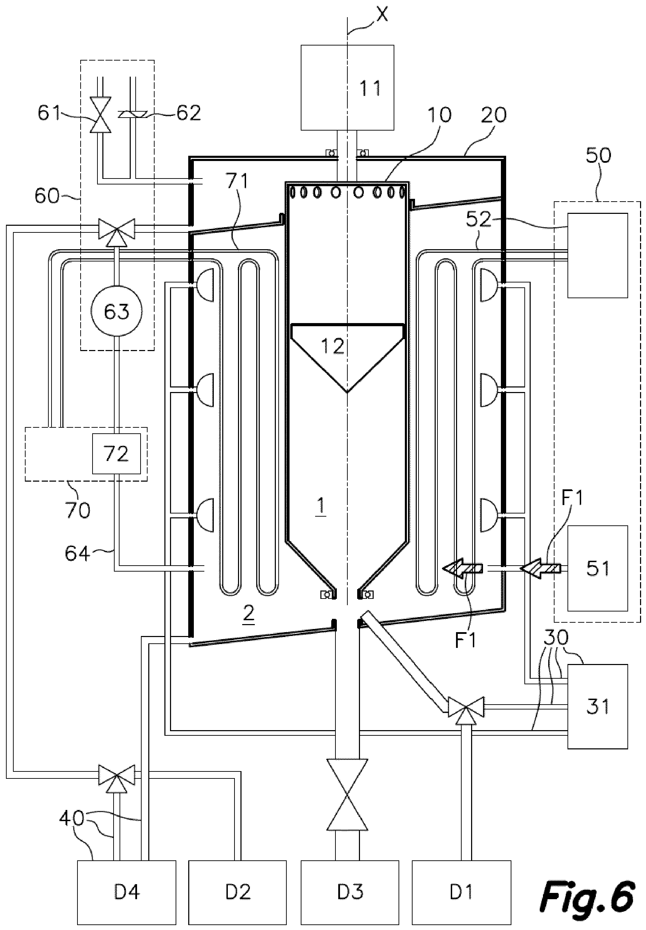

Fig. 6 shows the same embodiment shown in Fig. 1 but during the stand-by stage.

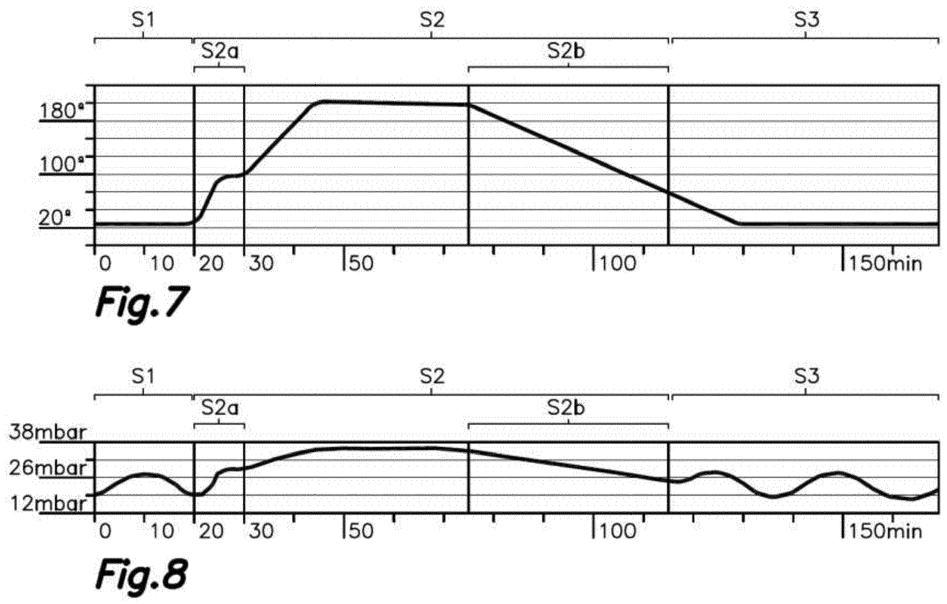

Fig. 7 shows a graph of the evolution of the temperature within the sealed housing during the cleaning stage, the sterilization stage, and the stand-by stage wherein the vertical axis represents the temperature in degrees Celsius, and the horizontal axis represents the time in minutes;
Fig. 8 shows a graph of the evolution of the pressure within the sealed housing during the cleaning stage, the sterilization stage, and the stand-by stage wherein the vertical axis represents the pressure in mbars, and the horizontal axis represents the time in minutes.

### Detailed description of an embodiment

The foregoing and other advantages and features will be more fully understood from the following detailed description of an embodiment with reference to the accompanying drawings, to be taken in an illustrative and not limitative.

According to a preferred embodiment, the automatic self-sterilizing supercentrifuge apparatus for liquid clarification includes a sealed housing 20 containing a tubular rotor 10 actuate by an actuation motor 11 to rotate around a vertical axis X.

The tubular rotor 10 defines a clarification chamber 1 in its interior.

The sealed housing 20 defines an intermediate chamber 2 in its interior, surrounding the tubular rotor 10.

The tubular rotor 10 is connected to the actuation motor 11 through a vertical shaft connected to an upper end of the tubular rotor 10. Said vertical shaft exits the sealed housing 20 through an air-tight bearing.

The lower end of the tubular rotor 10 includes a lower opening surrounded by a non-air-tight bearing contained within the sealed housing 20, allowing a communication between the intermediate chamber 2 and the clarification chamber 1 through said lower opening.

Said apparatus can perform a clarification stage, a cleaning stage S1, a sterilizing stage S2, and a stand-by stage S3.

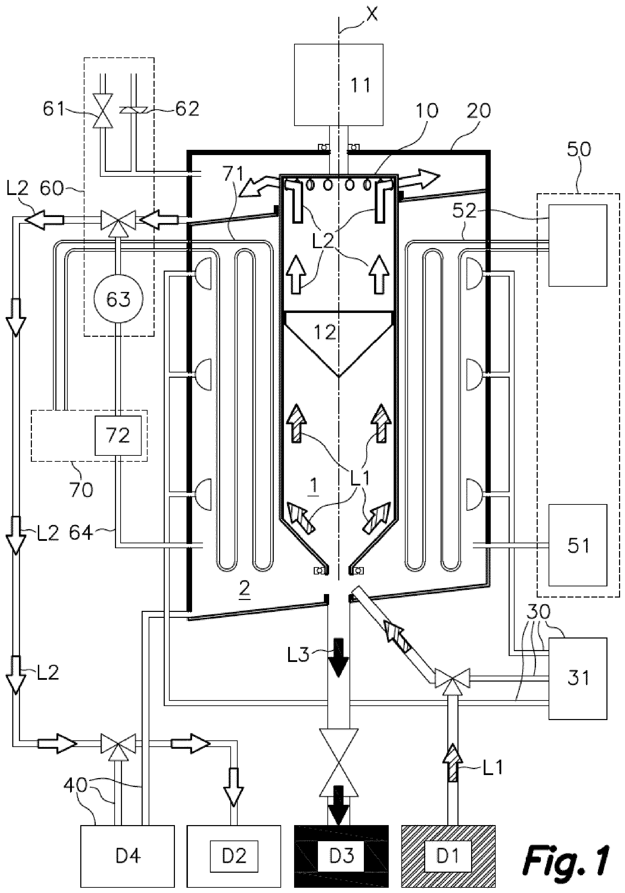

During the clarification stage, shown on Fig. 1, a liquid to be clarified L1, usually blood, is introduced in the tubular rotor 10 while it rotates, settling the solid fraction L3 and evacuating the liquid fraction L2 of said liquid to be clarified L1. The liquid to be clarified L1 is introduced within the tubular rotor 10 from a liquid to be clarifies source D1 from a feeding nozzle, said liquid to be clarified L1 being ejected in an upward inclined direction to the interior of the clarification chamber 1 though the lower opening of the tubular rotor 10.

The liquid fraction L2 is evacuated through openings if the upper end of the tubular rotor 10, collected in a collector and extracted from the sealed housing 20 through a pipe which conducts said clarified liquid L2 to a clarified liquid collector D2.

The solid fraction L3 is evacuated from the tubular rotor 10 through the lower opening, pushed downwards by a piston 12 contained within the tubular rotor 10 when the clarification has ended, the solid fraction L3 being evacuated through an evacuation conduct aligned with the lower opening of the tubular rotor 10 to a solid fraction collector D3.

The piston 12 will include valves to discretionally communicate the lower end and the upper end of the tubular rotor 10 through the piston 12.

When the clarification stage has ended, the interior of the clarification chamber 1 and of the intermediate chamber 2 has to be cleaned and sterilized.

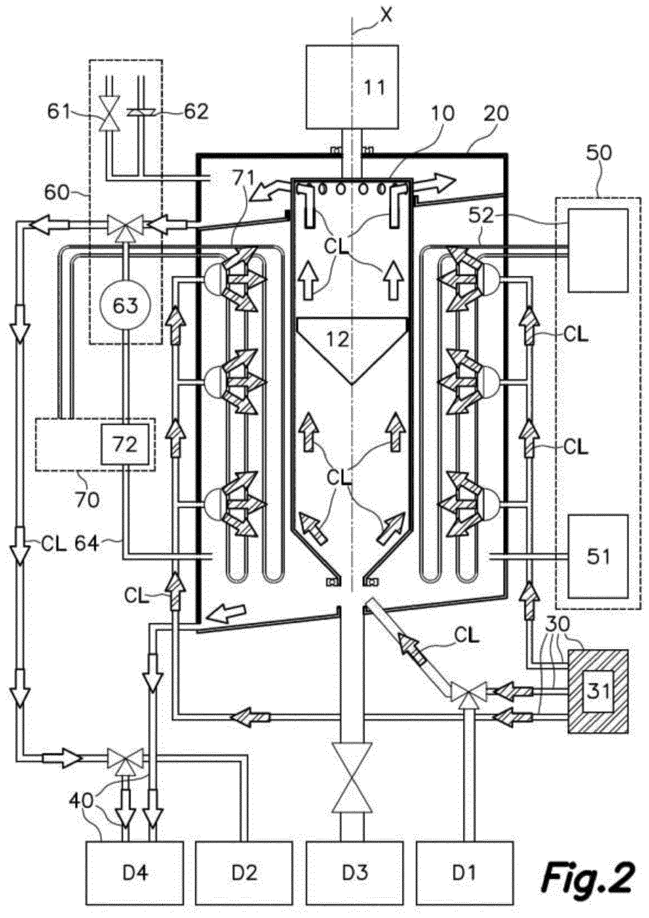

During the cleaning stage S1, shown on Fig. 2, a cleaning system 30 performs an automatic cleaning by introducing a cleaning liquid CL, provided by a cleaning liquid source 31, such clean water or purified water, in the clarification chamber 1, through the upper or the lower openings of the tubular rotor 10, preferably while is rotating, and in the intermediate chamber 2, preferably through nozzles, such sprinklers, contained in the intermediate chamber 2 and oriented towards the exterior surfaces of the tubular rotor 10.

Preferably the cleaning liquid CL is introduced into the clarification chamber 1 through the same feeding nozzle used to introduce the liquid to be clarified into the clarification chamber 1.

The cleaning system 30 further includes a cleaning liquid evacuation system 40 which evacuates the cleaning liquid CL from the clarification chamber 1 and from the intermediate chamber 2, preferably through the bottom end of those chambers to a cleaning liquid collector D4.

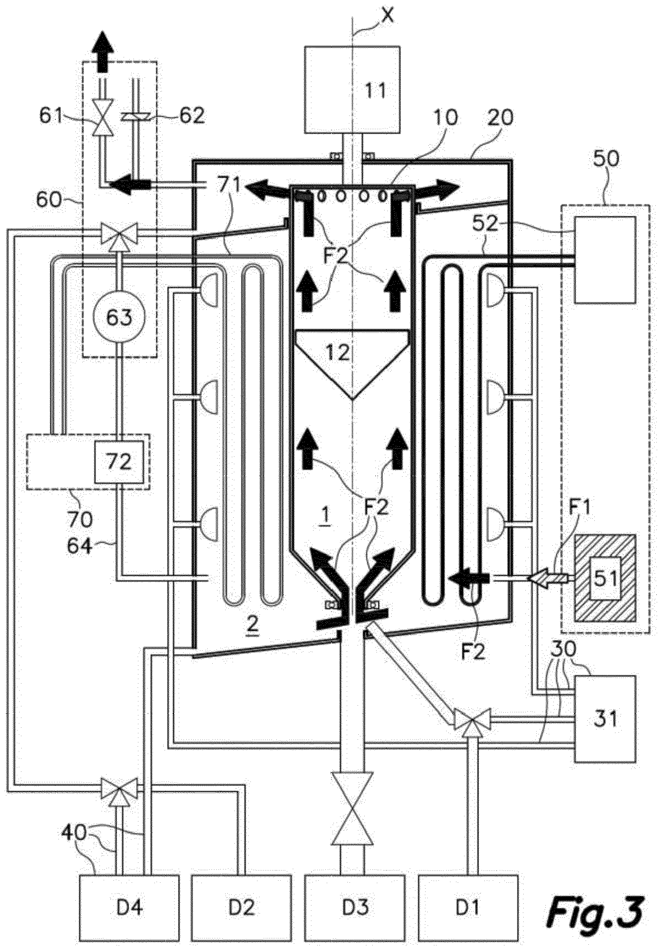

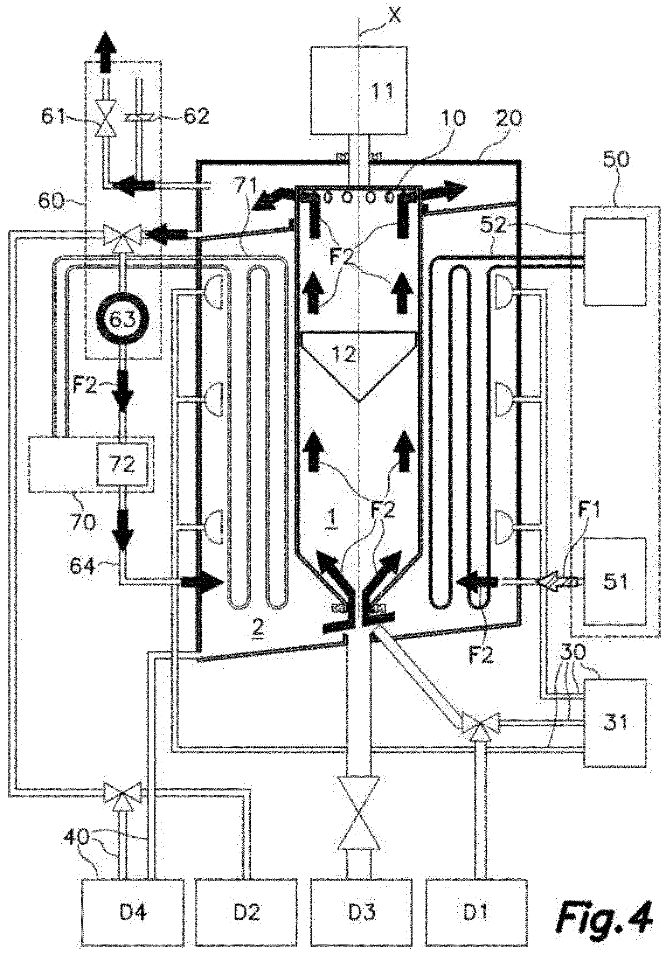

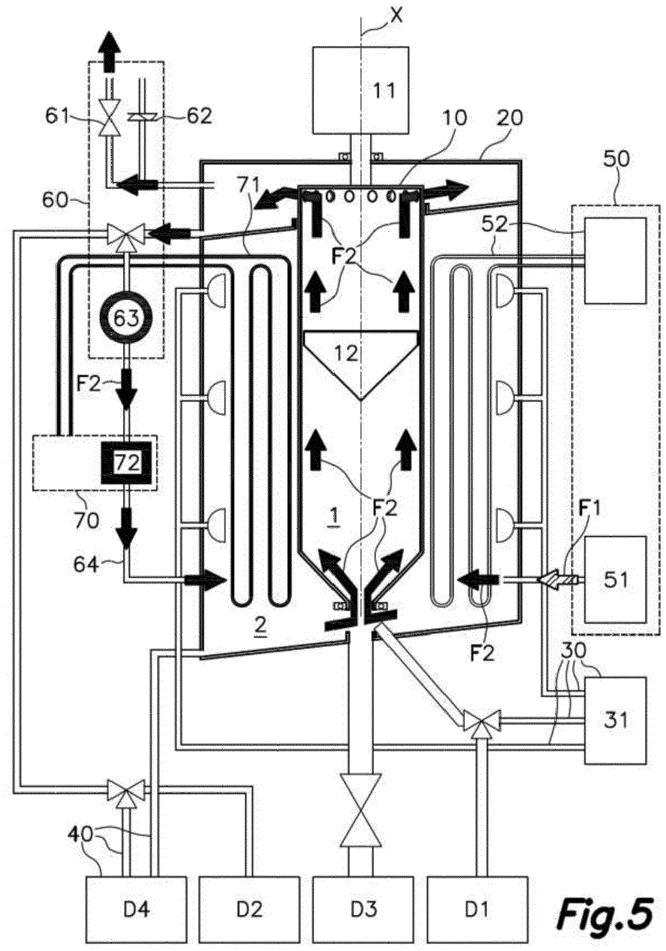

During a sterilization stage S2, shown on Figs. 3, 4 and 5, a sterilizing system 50 introduces sterilizing fluid F2 into the clarification chamber 1 and into the intermediate chamber 2 to perform an automatic sterilization thereof.

According to the preferred embodiment, the sterilizing system 50 comprises an air source providing pressurized filtered air to an air sterilization unit 51, which dry and sterilize the pressurized air producing dry sterilized air F1 at a pressure slightly above the ambient pressure. The dry sterilized air F1 is then introduced in the intermediate chamber 2, reaching the clarification chamber through the lower opening of the tubular rotor 10.

The sealed housing further contains an air heater 52, for example electrical heaters, which heats the dry sterilized air F1 to a temperature above 120°C, or above 150°C or above 180°C, transforming said dry sterilized air F1 into sterilizing fluid F2.

Preferably the sterilization stage S2 includes a drying stage S2a during the initial portion of the sterilization stage S2 immediately after the cleaning step S1, and a cooling stage S2b during the final portion of the sterilization stage S2, immediately before the stand-by stage S3.

During the drying stage S2a, shown on Fig. 3, the dry sterilized air F1 or the sterilizing fluid F2 contained within the intermediate chamber 2 and the clarification chamber 1 evaporates the remaining cleaning liquid CL and is then evacuated through a venting valve 61 of a sterilizing fluid evacuation system 60, drying the clarification chamber 1 and the intermediate chamber 2.

Once the drying stage S2a ends, when the drying has been completed, the introduction of sterilizing fluid F2 can be stopped or reduced, and the sterilizing fluid F2 already contained within the sealed housing 20 is maintained in the sealed housing 20, maintaining its temperature above 120°C and with a pressure slightly above the ambient pressure, during a period of time usually equal to or longer than 30 minutes.

If during this period the pressure drops below certain threshold, close to and above the minimal working pressure, additional dry sterilized air F1 can be introduced in the sealed housing 20 to increase its pressure. Equally, if the pressure increases above certain threshold, equal or above the maximal working pressure and below the pressure requiring a certificated pressure-containment-equipment, the pressure can be reduced through the venting valve 61. If the pressure reaches a pressure close to or equal to the pressure requiring a certificated pressure-containment-equipment and the venting valve 61 is unable to reduce the pressure, an emergency shear valve 62 will break to release the pressure.

According to a preferred embodiment, a flow of sterilizing fluid F2 is generated within the sealed housing 20 during the sterilizing step as part of said 30 minutes period of the sterilizing step.

Said flow is generated by suctioning sterilizing fluid F2 from the sealed housing 20 by an impeller, part of the sterilizing fluid evacuation system 60, and by reintroducing said sterilizing fluid F2 into the sealed housing 20 through a recirculation circuit 64.

Once the sterilization is completed, the temperature of the apparatus can be cooled down during the cooling stage S2b, for example, by turning off the air heater 52 and waiting until the temperature reaches the surrounding ambient temperature.

Alternatively, the apparatus can be actively cooled, through a cooling system 70, in an accelerated manner to reduce the time required to perform a new clarification step.

During initial moments of the cooling stage S2b the sterilizing fluid F2 passing through the recirculation circuit 64 can be cooled through a heat sink 72 integrated in said cooling system 70.

The heat sink 72 can be, for example, a cooling circuit feed with water or a heat exchanger in thermal contact with the sterilizing fluid F2 flowing through the recirculation circuit 64.

Once the temperature of the sealed housing 20 is under 100°C it can be directly cooled introducing cooling water in a coolant circuit 71 surrounding the sealed housing 20 and in thermal contact therewith.

The coolant circuit 71 is empty when the temperature of the sealed housing 20 is above 100°C and is filled with cooling water only when the temperature has been reduced under 100°C to prevent its boiling and the consequent increase in pressure.

## Claims

1. Automatic self-sterilizing supercentrifuge apparatus for liquid clarification including:
• a tubular rotor (10) actuated by an actuation motor (11) to rotate around a vertical axis (X), said tubular rotor (10) including a hollow interior defining a clarification chamber (1);
• a sealed housing (20) containing said tubular rotor (10), defining an intermediate chamber (2) between the tubular rotor (10) and the sealed housing (20);
• a cleaning system (30) including a cleaning liquid source (31) connected to the intermediate chamber (2) and to the clarification chamber (1) to provide a cleaning liquid (CL) thereto, and a cleaning liquid evacuation system (40) connected to the intermediate chamber (2) and to the clarification chamber (1) for evacuation of the cleaning liquid thereof;
• a sterilizing system (50) in communication with the intermediate chamber (2) and with the clarification chamber (1) and configured to generate a sterilizing fluid (F2) to sterilize the intermediate chamber (2) and the clarification chamber (1), and
• a sterilizing fluid evacuation system (60) in communication with the intermediate chamber (2) and with the clarification chamber (1) and configured to evacuate the sterilizing fluid (F2) thereof;
**characterized in that** the sterilizing system (50) includes:
• at least one air sterilization unit (51), configured to generate dry sterilized air (F1) with a moisture content below at least 10% and at least one air heater (52) configured to heat the dry sterilized air to at least 120°C to produce heated sterilized air as the sterilizing fluid (F2), the sterilizing system (50) being configured to provide the sterilizing fluid (F2) to the intermediate chamber (2) and to the clarification chamber (1) at a pressure comprised between a minimal working pressure, defined above the ambient pressure, and a maximal working pressure defined below a pressure requiring a certificated pressure-containment-equipment by regulations.

2. The automatic self-sterilizing supercentrifuge according to claim 1 wherein the minimal working pressure is equal or above 10mbars above the ambient pressure and/or wherein the maximal working pressure is below 500mbars above the ambient pressure, considered to be the pressure requiring a certificated pressure-containment-equipment, or below 200mbars above the ambient pressure, or preferably below 100mbars above the ambient pressure.

3. The automatic self-sterilizing supercentrifuge according to claim 1 or 2 wherein the clarification chamber (1) and the intermediate chamber (2) are in communication to each other permitting the free flow of the sterilizing fluid (F2), the sterilizing system (50) is connected to the intermediate chamber (2) and the sterilizing fluid evacuation system (60) is connected at least to the clarification chamber (1).

4. The automatic self-sterilizing supercentrifuge according to claim 1 or 2 wherein the sterilizing system (50) is connected to the intermediate chamber (2) and to the clarification chamber (1), and the sterilizing fluid evacuation system (60) is connected to the intermediate chamber (2) and to the clarification chamber (1).

5. The automatic self-sterilizing supercentrifuge according to any preceding claim wherein the sterilizing fluid evacuation system (60) include at least one venting valve (61) configured to evacuate sterilizing fluid (F2) from the clarification chamber (1) and/or from the intermediate chamber (2) when the pressure exceeds the maximum working pressure.

6. The automatic self-sterilizing supercentrifuge according to claim 5 wherein the sterilizing fluid evacuation system (60) further includes an emergency shear valve (62) configured to break under a pressure exceeding the maximum working pressure and lower than the pressure requiring a certificated-pressure-equipment.

7. The automatic self-sterilizing supercentrifuge according to any preceding claim wherein the sterilizing fluid evacuation system (60) is connected to an impeller (63), configured to suction the sterilizing fluid (F2) from the clarification chamber (1) and/or from the intermediate chamber (2) and to reintroduce at least part of said sterilizing fluid (F2) within the clarification chamber (1) and/or within the intermediate chamber (2) through a recirculation circuit (64), producing a flow of sterilizing fluid (F2) within the intermediate chamber (2) and/or the clarification chamber (1).

8. The automatic self-sterilizing supercentrifuge according to any preceding claim wherein the air heater (52) is contained within the intermediate chamber (2) and/or in thermal contact with the sealed housing (20), which is made of a metallic material.

9. The automatic self-sterilizing supercentrifuge according to claim 7 wherein the air heater (52) is a unit external to the sealed housing (20) and is connected with the air sterilization unit (51) and with the recirculation circuit (64) through electrovalves, said electrovalves being configured to conduct the dry sterilized air (F1) produced by the air sterilization unit (51) through the air heater (52) before reaching the intermediate chamber (2) and/or the clarification chamber (1) and to conduct the sterilizing fluid (F2), flowing through the recirculation circuit (64), through the air heater (52) before reaching the intermediate chamber (2) and/or the clarification chamber (1).

10. The automatic self-sterilizing supercentrifuge according to any preceding claim wherein the supercentrifuge further comprises a cooling system (70) including a coolant circuit (71) contained within the intermediate chamber (2) and/or in thermal contact with the sealed housing (20), which is made of a metallic material, and/or including a heat sink (72) in the recirculation circuit (64) configured to reduce the temperature of the sterilizing fluid (F2) passing there through.

11. Automatic self-sterilizing method for a supercentrifuge including a tubular rotor (10), with a hollow interior defining a clarification chamber (1), and a sealed housing (20) containing said tubular rotor (10), defining an intermediate chamber (2) between the tubular rotor (10) and the sealed housing (20), the method including the following steps:
• perform a cleaning stage by feeding a cleaning liquid into the intermediate chamber (2) and into the clarification chamber (1) and by evacuating said cleaning liquid from the intermediate chamber (2) and from the clarification chamber (1);
• perform a sterilizing stage by feeding a sterilizing fluid (F2) into the intermediate chamber (2) and into the clarification chamber (1), keeping the sterilizing fluid (F2) within the intermediate chamber (2) and the clarification chamber (1) for a period of time and later evacuating said sterilizing fluid (F2) therefrom;
**characterized in that**
• during the sterilizing stage the sterilizing fluid (F2) is generated by filtering, sterilizing and drying air, producing a dry sterilized air (F1) with a moisture content below 10% or below 5%, and by heating said dry sterilized air (F1) to a temperature above 120°C, and said sterilizing fluid (F2) is maintained into the clarification chamber (1) and into the intermediate chamber (2) at a pressure comprised between a minimal working pressure, defined above the ambient pressure, and a maximal working pressure defined below a pressure requiring a certificated pressure-containment-equipment by regulations.

12. The automatic self-sterilizing method according to claim 11 wherein the sterilization stage maintains the sterilizing fluid (F2) within the intermediate chamber (2) and the clarification chamber (1) at a temperature above 120°C during at least 30 minutes.

13. The automatic self-sterilizing method according to claim 11 or 12 wherein the sterilizing fluid (F2) is introduced in the intermediate chamber (2) and is later suctioned from the clarification chamber (1), producing a flow of sterilizing fluid (F2) from the intermediate chamber (2) to the clarification chamber (1), all or part of the sterilizing fluid (F2) being later reintroduced in the intermediate chamber (2).

14. The automatic self-sterilizing method according to claim 13 wherein the sterilization stage include a cool down stage during which the sealed housing (20) is actively cooled by pumping a coolant through a coolant circuit (71), of a cooling system (70), contained within the intermediate chamber (2) and/or in thermal contact with the sealed housing (20), which is made of a metallic material, and/or by pumping a coolant through a heat sink (72) in the recirculation circuit (64) configured to reduce the temperature of the sterilizing fluid (F2) passing there through.

15. The automatic self-sterilizing method according to any preceding claim 11 to 14 wherein, after the sterilization stage, a stand-by stage is performed maintaining the dry sterilized air (F1) at ambient temperature within the clarification chamber (1) and the intermediate chamber (2) at the pressure comprised between the minimal working pressure, defined above the ambient pressure, and the maximal working pressure defined below a pressure requiring a certificated pressure-containment-equipment by regulations.
